(19) **Europäisches Patentamt / European Patent Office / Office européen des brevets**

(11) **EP 3 706 436 B1**

(12) **EUROPEAN PATENT SPECIFICATION**

(45) Date of publication and mention of the grant of the patent:
**27.09.2023 Bulletin 2023/39**

(51) International Patent Classification (IPC):
**H04R 17/00** (2006.01)   **A61B 8/00** (2006.01)
**H04R 31/00** (2006.01)   **B06B 1/06** (2006.01)
**C08L 63/00** (2006.01)   **G10K 11/02** (2006.01)

(21) Application number: **18871994.2**

(52) Cooperative Patent Classification (CPC):
(C-Sets available)
**G10K 11/02; A61B 8/4281; A61B 8/4444;
B06B 1/067; C08L 63/00**      (Cont.)

(22) Date of filing: **31.10.2018**

(86) International application number:
**PCT/JP2018/040441**

(87) International publication number:
**WO 2019/088148 (09.05.2019 Gazette 2019/19)**

(54) **RESIN COMPOSITION FOR ACOUSTIC MATCHING LAYER, HARDENED PRODUCT, ACOUSTIC MATCHING SHEET, ACOUSTIC WAVE PROBE, ACOUSTIC WAVE MEASUREMENT DEVICE, ACOUSTIC WAVE PROBE PRODUCTION METHOD**

HARZZUSAMMENSETZUNG FÜR AKUSTISCHE ANPASSUNGSSCHICHT, GEHÄRTETES PRODUKT, AKUSTISCHE ANPASSUNGSFOLIE, AKUSTISCHE WELLENSONDE, AKUSTISCHE WELLENMESSVORRICHTUNG, VERFAHREN ZUR HERSTELLUNG DER AKUSTISCHEN WELLENSONDE

COMPOSITION DE RÉSINE POUR COUCHE D'ADAPTATION ACOUSTIQUE, PRODUIT DURCI, FEUILLE D'ADAPTATION ACOUSTIQUE, SONDE D'ONDE ACOUSTIQUE, DISPOSITIF DE MESURE D'ONDE ACOUSTIQUE, PROCÉDÉ DE PRODUCTION DE SONDE D'ONDE ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2017   JP 2017212208**

(43) Date of publication of application:
**09.09.2020 Bulletin 2020/37**

(73) Proprietor: **FUJIFILM Corporation**
**Tokyo 106-8620 (JP)**

(72) Inventors:
• **NAKAI, Yoshihiro**
  **Ashigarakami-gun**
  **Kanagawa 258-8577 (JP)**
• **HAMADA, Kazuhiro**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-B1- 2 004 064          JP-A- H0 462 007
JP-A- H11 113 908         JP-A- 2001 200 169
JP-A- 2009 296 055        JP-A- 2010 013 326
JP-A- 2012 034 160        JP-A- 2013 081 241
US-A1- 2016 199 031      US-A1- 2016 338 666
US-A1- 2017 252 465**

(52) Cooperative Patent Classification (CPC): (Cont.)

C-Sets
**C08L 63/00, C08K 3/08;**
**C08L 63/00, C08K 9/02**

C-Sets
**C08L 63/00, C08K 3/08;**
**C08L 63/00, C08K 9/02**

**Description**

**BACKGROUND OF THE INVENTION**

1. Field of the Invention

The present invention relates to resin compositions for acoustic matching layers, cured products, acoustic matching sheets, acoustic wave probes, acoustic wave measuring apparatuses and methods for manufacturing acoustic wave probes.

2. Description of the Related Art

Acoustic wave measuring apparatuses include an acoustic wave probe configured to irradiate a subject such as a living body with an acoustic wave and to receive the reflected wave (echo) and output a signal. The reflected wave received by the acoustic wave probe is converted into an electrical signal and is displayed as an image. Thus, the acoustic wave probe can be used to visualize and examine the inside of the subject.

Acoustic waves such as ultrasound and photoacoustic waves are selected as appropriate depending on the subject and the measurement conditions.

For example, ultrasound diagnostic apparatuses, which are a type of acoustic wave measuring apparatus, transmit ultrasound toward the inside of the subject, receive the ultrasound reflected by the tissue inside the subject, and display it as an image.

Photoacoustic wave measuring apparatuses, on the other hand, receive an acoustic wave radiated from inside the subject by a photoacoustic effect and display it as an image. The photoacoustic effect is a phenomenon in which, when the subject is irradiated with a pulsed electromagnetic wave such as visible light, near-infrared light, or microwave, the subject absorbs the electromagnetic wave to generate heat that causes the subject to expand thermally, thereby generating an acoustic wave (typically ultrasound).

For acoustic wave measuring apparatuses to transmit and receive an acoustic wave to and from the subject, the acoustic impedance of the acoustic wave probe needs to match that of the subject. To meet this need, the acoustic wave probe is provided with an acoustic matching layer. To explain this, a probe for an ultrasound diagnostic apparatus (also referred to as "ultrasound probe"), which is a type of acoustic wave probe, will be described by way of example.

An ultrasound probe includes a piezoelectric element configured to transmit and receive ultrasound, an acoustic lens for contact with a living body, and an acoustic matching layer disposed between the piezoelectric element and the acoustic lens. The ultrasound transmitted from the piezoelectric element travels through the acoustic matching layer and then through the acoustic lens to enter the living body. There is generally a difference in acoustic impedance (density × sound velocity) between the acoustic lens and the living body. As the difference becomes larger, the ultrasound is more likely to be reflected by the surface of the living body, which decreases the efficiency with which the ultrasound enters the living body. Thus, the acoustic impedance characteristics of the acoustic lens need to be close to those of the living body.

On the other hand, there is generally a large difference in acoustic impedance between the piezoelectric element and the living body. Accordingly, there is also generally a large difference in acoustic impedance between the piezoelectric element and the acoustic lens. Thus, if the piezoelectric element and the acoustic lens are stacked together, the ultrasound generated from the piezoelectric element is reflected at the interface between the piezoelectric element and the acoustic lens, which decreases the efficiency with which the ultrasound enters the living body. To reduce the reflection of the ultrasound at the interface, the acoustic matching layer mentioned above is provided between the piezoelectric element and the acoustic lens. The acoustic impedance of the acoustic matching layer lies between the acoustic impedance of the living body or the acoustic lens and the acoustic impedance of the piezoelectric element so that the ultrasound propagates efficiently from the piezoelectric element into the living body. It is also known to form an acoustic matching layer having a multilayer structure to provide a stepwise gradient in acoustic impedance from the piezoelectric element side toward the acoustic lens side so that the ultrasound propagates more efficiently.

In addition to the acoustic characteristics described above, acoustic wave probes need to have sufficient mechanical strength. That is, the mechanical strength directly affects the product life of acoustic wave probes because they are moved in contact with and occasionally pressed against a living body during use.

It is known to use a silicone resin as a constituent material for an acoustic lens. Silicone resins, without any modification, have low density and differ greatly in acoustic impedance from a living body. Silicone resins also tend to be soft and have poor mechanical strength when used alone.

As a technique for addressing this problem, for example, JP2017-12436A discloses a silicone resin, for an acoustic wave probe, that has a particular structure and that is mixed with inorganic oxide particles having a particular particle size. The technique disclosed in JP2017-12436A reportedly provides an acoustic wave probe whose acoustic impedance is close to that of a living body, that exhibits reduced acoustic wave attenuation, and that has good mechanical strength.

On the other hand, it is known to use a silicone resin as a constituent material for an acoustic matching layer, and it is also known to use a material other than a silicone resin. For example, JP2013-192113A discloses an acoustic wave matching layer, for an ultrasound probe, formed by firing a composition containing metal nanoparticles having particle sizes of 1 $\mu$m or less and an inorganic polymer binder serving as a binder at low temperature so that the composition is partially or completely converted into a bulk of metal. The technique disclosed in JP2013-192113A reportedly allows an acoustic matching layer having the desired acoustic impedance characteristics to be produced. US 2017/252465 A1 describes a composition for an acoustic wave probe, comprising a polysiloxane mixture containing polysiloxane having a vinyl group, polysiloxane having two or more Si-H groups in a molecular chain, and one or more inorganic compound particles, wherein the average primary particle diameter of the inorganic compound particles is less than 25 nm and the inorganic compound particles are selected from the group consisting of magnesium oxide, titanium oxide, iron oxide, zinc oxide, zirconium oxide, barium oxide, tin oxide, and ytterbium oxide. JP 2009 296055 A describes an acoustic matching layer for an ultrasonic probe. US 2016/338666 A1 describes an acoustic lens for an ultrasound probe comprising a vulcanized molded rubber composition containing silicon rubber and metal oxide particles dispersed in the silicon rubber. JP H04 62007 A describes a resin composite material comprising a resin and a ferromagnetic metal powder whose surface is uniformly covered with a thin film of an inorganic metal phosphate compound containing no iron and surface-treated with an isocyanate compound. JP 2001 200169 A describes a resin composite material comprising ferromagnetic metal powder whose surface is coated with a thin film of an inorganic phosphate metal compound not containing an organic group. EP 2 004 064 A2 describes matching layers for an ultrasonic transducer stack having a matching layer comprising a matrix material loaded with a plurality of micron-sized and nano-sized particles.

## SUMMARY OF THE INVENTION

The acoustic matching layer of an acoustic wave probe is formed from a material whose acoustic impedance can be adjusted to the desired level between the acoustic impedance of the piezoelectric element and the acoustic impedance of the living body or the acoustic lens.

One such acoustic matching layer that has been reported is the acoustic matching layer disclosed in JP2013-192113A above. However, the composition used to form the acoustic matching layer disclosed in JP2013-192113A contains a large amount of metal nanoparticles and thus has high viscosity, which results in, for example, poor handleability and workability. In addition, metals are susceptible to corrosion under reducing conditions and are therefore disadvantageous in terms of, for example, probe maintenance and life.

In particular, as described above, if an acoustic matching layer has a multilayer structure, the acoustic matching layer needs to be subjected to secondary processing such as cutting or dicing to a thickness level of several hundreds of micrometers or less during the production process. Accordingly, in addition to the mechanical strength generally required of acoustic wave probes as described above, the constituent material for the acoustic matching layer needs to have high mechanical strength from the viewpoint of secondary processing. However, research conducted by the inventors has revealed that it is difficult to achieve the desired mechanical strength with the metal particles disclosed in JP2013-192113A above.

Accordingly, an object of the present invention is to provide a resin composition, for an acoustic matching layer, that contains metal particles and a binder including a resin, that, if the resin is thermoplastic, exhibits good fluidity (handleability) upon melting of the resin even if the resin composition contains a large amount of metal particles, and if the resin is thermosetting, exhibits good fluidity before curing even if the resin composition contains a large amount of metal particles, and that can be formed or processed into the desired sheet shape to form an acoustic matching layer having good mechanical strength and corrosion resistance and little variation in acoustic characteristics in the layer. Another object of the present invention is to provide a material set, for an acoustic matching layer, that is suitable for preparation of such a composition.

Another object of the present invention is to provide an acoustic matching sheet that has good corrosion resistance and mechanical strength and little variation in acoustic characteristics in the sheet even though the sheet contains metal particles, and also to provide a cured product that can be used for such an acoustic matching sheet.

Another object of the present invention is to provide an acoustic wave probe that has good corrosion resistance and mechanical strength and little variation in acoustic characteristics in the probe even though the acoustic matching layer contains metal particles, and also to provide an acoustic wave measuring apparatus including such an acoustic wave probe.

Another object of the present invention is to provide a method for manufacturing an acoustic wave probe by which an acoustic wave probe can be manufactured that has good corrosion resistance and mechanical strength and little variation in acoustic characteristics in the probe even though the acoustic matching layer contains metal particles.

After conducting intensive research in view of the foregoing problems, the inventors have found that the use of a mixture of surface-treated metal particles and a binder including a resin (also referred to as "matrix" or "dispersion medium") as a composition for forming an acoustic matching layer increases the fluidity of the composition to the desired level even if the composition contains a large amount of metal particles, thus providing a composition having good handleability. A sheet formed from the composition has also been found to have good corrosion resistance and mechanical strength and little variation in acoustic characteristics in the sheet even though the sheet contains metal particles.

After conducting further research based on these findings, the inventors have completed the present invention. A resin composition for an acoustic matching layer according to the present invention is defined in the claims.

The resin composition for an acoustic matching layer and the material set for an acoustic matching layer according to the present invention exhibit good fluidity even if they contain a large amount of metal particles, and can also be formed or processed into the desired sheet shape to form an acoustic matching sheet having good mechanical strength and corrosion resistance and little variation in acoustic characteristics in the sheet.

In addition, the acoustic matching sheet according to the present invention has good corrosion resistance and mechanical strength and little variation in acoustic characteristics in the sheet even though the sheet contains metal particles. In addition, the cured product according to the present invention is suitable for use as a constituent material for the acoustic matching layer according to the present invention.

In addition, the acoustic wave probe and the acoustic wave measuring apparatus including the acoustic wave probe according to the present invention have good corrosion resistance and mechanical strength and little variation in acoustic characteristics in the probe even though the acoustic matching layer contains metal particles.

In addition, the method for manufacturing an acoustic wave probe according to the present invention provides an acoustic wave probe having good corrosion resistance and mechanical strength and little variation in acoustic characteristics in the probe even though the acoustic matching layer contains metal particles.

## BRIEF DESCRIPTION OF THE DRAWINGS

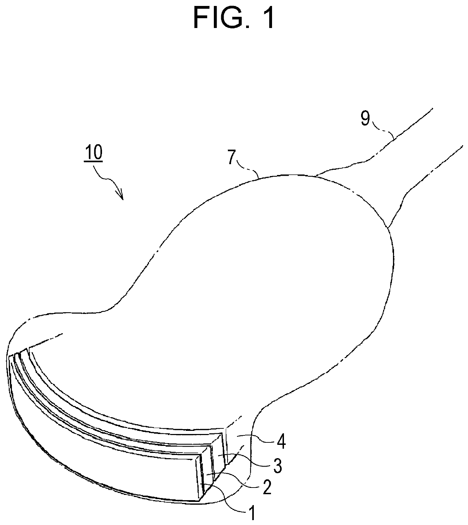

FIG. 1 is a perspective see-through view illustrating an example convex ultrasound probe as one form of acoustic wave probe.

## DESCRIPTION OF THE PREFERRED EMBODIMENTS

Resin Composition for Acoustic Matching Layer

A resin composition for an acoustic matching layer according to the present invention (hereinafter also simply referred to as "the composition according to the present invention") includes a binder including a resin and surface-treated metal particles. Binder

The binder present in the composition according to the present invention fills the gaps between the surface-treated metal particles and functions as a dispersion medium for the surface-treated metal particles.

The binder includes a thermoplastic resin or a thermosetting resin

If a thermoplastic resin is used as the binder, the thermoplastic resin preferably has a melting point of 100°C to 350°C. If the binder is a thermoplastic resin, i.e., if the composition according to the present invention has a thermoplastic resin as the binder, the thermoplastic resin is solid in a temperature range below the melting point of the thermoplastic resin, and the surface-treated metal particles are dispersed in the solidified resin.

The thermoplastic resin that can be used as the binder may be any thermoplastic resin. Preferred examples of thermoplastic resins include polyamide resins, acrylonitrile-butadiene-styrene copolymers, meth(acrylic) resins, polyacetal resins, polycarbonate resins, polyethersulfone resins, polyetherimide resins, thermoplastic polyurethane resins, polyester resins (such as polyethylene terephthalate and polybutylene terephthalate), polyolefin resins (such as polyethylene and polypropylene), polyvinyl resins (such as polyvinyl chloride), polyetheretherketone resins, polyphenylene sulfide resins, thermoplastic polyimide resins, polyamide-imide resins, polystyrene resins, and fluorocarbon resins (such as polytetrafluoroethylene and polyvinylidene fluoride). These thermoplastic resins can be used alone or in combination.

Particularly preferred are polyamide resins, acrylonitrile-butadiene-styrene copolymers, meth(acrylic) resins, polyacetal resins, polycarbonate resins, polyethersulfone resins, polyetherimide resins, and thermoplastic polyurethane resins. The use of these thermoplastic resins allows the composition to exhibit a higher fluidity when the resin is in a molten state. Thus, for example, good handleability and workability can be achieved at a higher level.

According to the invention the binder includes epoxy resins, phenolic resins, melamine resins, urea resins, unsaturated polyester resins, alkyd resins, thermosetting polyurethane resins, and thermosetting polyimide resins.

In particular, it is preferred to use an epoxy resin as the thermosetting resin that forms the binder to increase the crosslink density and thereby further improve the mechanical strength of the resulting sheet. In this case, the binder preferably includes an epoxy resin in combination with a curing agent. That is, if the binder includes a thermosetting resin, the binder is preferably a combination of an epoxy resin and a curing agent.

If an epoxy resin is used as the thermosetting resin, the epoxy resin preferably includes at least one of a bisphenol A epoxy resin, a bisphenol F epoxy resin, or a phenol novolac epoxy resin.

The bisphenol A epoxy resin that can be used in the present invention may be any bisphenol A epoxy resin, and a wide range of bisphenol A epoxy resins that are commonly used as base resins for epoxy adhesives can be used. Specific preferred examples of such bisphenol A epoxy resins include bisphenol A diglycidyl ethers (such as jER825, jER828, and jER834 (all trade names) available from Mitsubishi Chemical Corporation) and bisphenol A propoxylate diglycidyl ethers (such as those available from Sigma-Aldrich Company).

The bisphenol F epoxy resin that can be used in the present invention may be any bisphenol F epoxy resin, and a wide range of bisphenol F epoxy resins that are commonly used as base resins for epoxy adhesives can be used. Specific preferred examples of such bisphenol F epoxy resins include bisphenol F diglycidyl ethers (such as the trade name EPICLON 830 available from DIC Corporation) and 4,4'-methylenebis(N,N-diglycidylaniline).

The phenol novolac epoxy resin that can be used in the present invention may be any phenol novolac epoxy resin, and a wide range of phenol novolac epoxy resins that are commonly used as base resins for epoxy adhesives can be used. An example of such a phenol novolac epoxy resin is one available as Product No. 406775 from Sigma-Aldrich Company.

The curing agent may be any compound known for use as a curing agent for epoxy resins. Examples of such compounds include aliphatic amines, aromatic amines, dicyandiamide, dihydrazide compounds, acid anhydrides, and phenolic resins.

It is preferred to use at least one of a primary amine or a secondary amine to increase the crosslink density and thereby further improve the mechanical strength of the resulting sheet. Particularly preferred are compounds having primary and secondary amines in one molecule, specific examples of which include polyamidoamines and triethylenetetramine.

If the composition according to the present invention includes an epoxy resin and a curing agent as the binder, the curing reaction of the epoxy resin in the composition may proceed over time even under mild conditions. Thus, the properties of the composition may change over time and may therefore be unstable. However, for example, if the composition is stored at a temperature of -10°C or lower, the curing reaction does not occur or is sufficiently inhibited, and the individual components can be stably maintained in the composition.

If an epoxy resin is used as the binder, a material set for an acoustic matching layer is also preferred that separately includes a resin composition including an epoxy resin and surface-treated metal particles, serving as a base resin, and a curing agent. When an acoustic matching layer is formed, the base resin and the curing agent are mixed together to prepare the composition according to the present invention before the composition is used to form a layer. Thus, an acoustic matching layer can be formed.

The mass ratio of the epoxy resin forming the binder to the curing agent may be adjusted as appropriate depending on, for example, the type of curing agent used. For example, the ratio of the epoxy resin to the curing agent may be 99/1 to 20/80, preferably 90/10 to 40/60.

If the material set for an acoustic matching layer mentioned above is used to prepare the composition according to the present invention by mixing together the base resin and the curing agent before layer formation, the base resin and the curing agent are preferably mixed together such that the mass ratio of the epoxy resin to the curing agent is 99/1 to 20/80, more preferably such that the mass ratio of the epoxy resin to the curing agent is 90/10 to 40/60.

Surface-Treated Metal Particles

The composition according to the present invention contains metal particles subjected to surface treatment (surface-treated metal particles). By adjusting the amount of surface-treated metal particles present in the composition, the density of the composition can be adjusted, and accordingly, the acoustic impedance of the resulting acoustic matching layer can be adjusted to the desired level.

In addition, if surface-treated metal particles are used, the composition exhibits sufficient fluidity even if the composition contains a large amount of metal particles. This results in good handleability and workability. Furthermore, a sheet formed from the composition has good mechanical strength. Although the mechanism is not fully understood, these are attributable to the combined effect of weakened aggregation force between the metal particles and increased affinity between the metal particles and the resin due to surface treatment.

The surface treatment according to the present invention is as defined in the claims. The metal particles may be surface-treated by any technique, and common surface treatment techniques can be used. Examples of such techniques useful for understanding the invention include treatment with oils such as hydrocarbon oils, ester oils, and lanolin; treatment with silicones such as dimethyl polysiloxane, methyl hydrogen polysiloxane, and methyl phenyl polysiloxane; treatment with fluorine compounds such as perfluoroalkyl-containing esters, perfluoroalkylsilanes, perfluoropolyethers, and perfluoroalkyl-containing polymers; treatment with silane coupling agents such as 3-methacryloxypropyltrimethoxysilane and 3-glycidoxypropyltrimethoxysilane; treatment with titanium coupling agents such as isopropyltriisostearoyl titanate and isopropyl tris(dioctylpyrophosphate) titanate; metallic soap treatment; treatment with amino acids such as acylglutamic acids; treatment with lecithins such as hydrogenated egg yolk lecithin; collagen treatment; polyethylene treatment; moisture retention treatment; inorganic compound treatment; mechanochemical treatment; and treatment with phosphoric acid compounds such as phosphoric acid, phosphorous acid, phosphate salts, and phosphite salts.

The surface treatment is in one embodiment of the invention phosphoric acid compound treatment (treatment with a phosphoric acid compound), as further defined in claim 1. By phosphoric acid compound treatment, a thick, highly polar treated layer can be formed on the surface of the metal particles. As a result, the surface of the metal particles can be effectively modified. Specifically, the corrosion resistance of the metal particles can be effectively increased, and an increase in the viscosity of the composition can be more effectively inhibited because of increased affinity with the binder and reduced hydrophobic aggregation of the metal particles. In addition, for example, because the metal particles are evenly dispersed in the binder, the mechanical strength of the sheet formed from the composition can be further increased, and there is less variation in acoustic characteristics.

According to the invention, the phosphoric acid compound is iron phosphate. Other Examples of phosphoric acid compounds useful for understanding the invention include phosphoric acid, phosphorous acid, hypophosphorous acid, pyrophosphoric acid, linear polyphosphoric acid, cyclic metaphosphoric acid, and salts thereof. If the phosphoric acid compound is in salt form, a metal salt is preferred. Examples of metal salts include, but not limited to, alkali metals, alkaline earth metals, and ammonium salts.

The phosphoric acid compounds listed above can be used alone or in combination for surface treatment. These phosphoric acid compounds are typically mixed with additives such as chelating agents and neutralizers for use as surface treatment agents. Commercially available aqueous solutions of phosphoric acid compounds can also be used as surface treatment agents.

The phosphoric acid compound treatment can be performed, for example, by mixing the metal particles with a surface treatment agent including a phosphoric acid compound as listed above. The conditions such as mixing time and temperature may be set as appropriate depending on the purpose. In this treatment, the dissociation (equilibrium) reaction of the phosphoric acid compound is utilized to precipitate an insoluble phosphoric acid compound on the surface of the metal particles.

For phosphoric acid compound treatment, reference may be made to, for example, "Journal of the Surface Finishing Society of Japan", Vol. 61, No. 3, p. 216, 2010, or "Journal of the Surface Finishing Society of Japan", Vol. 64, No. 12, p. 640, 2013.

From the viewpoint of sensitivity, surface treatment with a silane coupling agent (silane coupling agent treatment) is preferred. Although the exact mechanism is not fully understood, it is believed that surface treatment with a silane coupling agent allows for less ultrasound attenuation than other types of surface treatment because silane coupling agent treatment increases the interaction between the metal and the binder and thus reduces reflection and scattering of ultrasound at the interface between the metal particles and the binder.

The metal forming the surface-treated metal particles may be any metal. A metal atom may be used alone or in the form of a metal carbide, nitride, oxide, or boride. The metal may also be alloyed. Examples of alloys include high-tensile-strength steel (Fe-C), chromium-molybdenum steel (Fe-Cr-Mo), manganese-molybdenum steel (Fe-Mn-Mo), stainless steel (Fe-Ni-Cr), Alloy 42, invar (Fe-Ni), permendur (Fe-Co), silicon steel (Fe-Si), red brass, tombac (Cu-Zn), nickel-silver (Cu-Zn-Ni), bronze (Cu-Sn), cupronickel (Cu-Ni), shakudo (Cu-Au), constantan (Cu-Ni), duralumin (Al-Cu), Hastelloy (Ni-Mo-Cr-Fe), Monel (Ni-Cu), Inconel (Ni-Cr-Fe), nichrome (Ni-Cr), ferromanganese (Mn-Fe), and cemented carbide (WC/Co).

The metal forming the surface-treated metal particles preferably includes at least one metal in Groups 4 to 12 of the periodic table (preferably consists of at least one metal in Groups 4 to 12 of the periodic table). The metal in Groups 4 to 12 of the periodic table is preferably a metal in Period 4 of the periodic table. The use of a metal in Period 4 of the periodic table increases the affinity of the metal particles with the binder, further improves the corrosion resistance, and effectively contributes to improved mechanical strength of the resulting sheet.

More preferably, the metal includes at least one of iron, zinc, titanium, copper, or nickel (preferably consists of at least one of iron, zinc, titanium, copper, or nickel). Even more preferably, the metal includes at least one of iron, copper, or nickel (preferably consists of at least one of iron, copper, or nickel).

The metal preferably includes no oxide form.

To reduce the viscosity of the resin composition and to reduce acoustic attenuation, the surface-treated metal particles used in the present invention preferably have a particle size of 1 to 10 $\mu$m. As used herein, the "particle size" of the surface-treated metal particles refers to the average primary particle size.

As used herein, "average primary particle size" refers to the volume average particle size. The volume average particle size is determined as follows.

The surface-treated metal particles are added to methanol to a concentration of 0.5% by mass and are dispersed by sonication for 10 minutes. The particle size distribution of the thustreated surface-treated metal particles is measured with a laser diffraction/scattering particle size distribution analyzer (the trade name LA-950V2 available from Horiba, Ltd.), and the volume-based median size thereof is determined as the volume average particle size. The median size corresponds to a cumulative volume of 50% when the particle size distribution is represented as a cumulative distribution.

In the surface-treated metal particles, the surface-treated layer covering the metal particles preferably has a thickness of 1 to 1,000 nm, more preferably 5 to 100 nm.

The surface-treated layer need not cover the entire surface of the metal particles, but may be partially missing as long as it does not interfere with the advantages of the present invention.

The amounts of surface-treated metal particles and binder present in the composition according to the present invention are adjusted as appropriate depending on, for example, the target acoustic impedance. For example, if an acoustic matching layer composed of a plurality of layers is formed, the composition used for the acoustic matching layer on the piezoelectric element side may contain a relatively large amount of surface-treated metal particles, whereas the composition used for the acoustic matching layer on the acoustic lens side may contain a relatively small amount of surface-treated metal particles. This provides a gradient in acoustic impedance from the piezoelectric element side toward the acoustic lens side so that an acoustic wave propagates more efficiently.

The amount of binder present in the composition according to the present invention may be, for example, 10 to 80 parts by mass, more preferably 15 to 75 parts by mass, even more preferably 20 to 70 parts by mass, based on 100 parts by mass of the surface-treated metal particles.

The composition according to the present invention is composed of the binder and the surface-treated metal particles. The composition according to the present invention may further contain other components as long as they do not interfere with the advantages of the present invention. Examples of components other than the binder and the surface-treated metal particles include curing retarders, solvents, dispersing agents, pigments, dyes, antistatic agents, antioxidants, flame retardants, and thermal conductivity improvers.

The total amount of binder and surface-treated metal particles present in the composition according to the present invention is preferably 80% by mass or more, more preferably 90% by mass or more.

If the binder is a thermoplastic resin, the composition according to the present invention preferably has a viscosity of 1 to 5,000 Pa s, more preferably 10 to 1,000 Pa s, at a temperature of 20°C above the melting point of the resin. If two or more thermoplastic resins are used, "20°C above the melting point of the resin" refers to "20°C above the melting point of the resin having the highest melting point".

Alternatively, if the binder is a thermosetting resin, the composition according to the present invention preferably has a viscosity of 1 to 5,000 Pa s, more preferably 10 to 1,000 Pa·s, at 25°C before curing (within 60 minutes after the thermosetting resin and the curing agent are homogeneously mixed together at 25°C). For a material set for an acoustic matching layer according to the present invention, it is preferred that the viscosity of the base resin at 25°C fall within the above preferred range.

The viscosity is measured with a cone-type rotational rheometer ("RheoStress RS6000" available from Haake Inc.) under the following conditions: a temperature of 25°C, 1 rpm, and a measurement time of 60 seconds.

Preparation of Resin Composition for Acoustic Matching Layer

The resin composition for an acoustic matching layer according to the present invention can be prepared, for example, by mixing together the constituent components for the resin composition for an acoustic matching layer. Mixing may be performed by any technique by which the components can be homogeneously mixed together, for example, using an agitator or by kneading with a mixer (such as a kneader, a pressure kneader, a Banbury mixer (continuous kneader), or a two-roll mill). If a thermoplastic resin is used as the binder, the components are kneaded at a temperature higher than or equal to the melting point of the resin. Thus, a resin composition, for an acoustic matching layer, in which the surface-treated metal particles are dispersed in the binder can be prepared.

Alternatively, if a thermosetting resin is used as the binder, a composition, for an acoustic matching layer, in which the surface-treated metal particles are dispersed in the binder can be prepared by mixing together the uncured binder and the surface-treated metal particles and optionally further mixing a curing agent. In this case, although the components may be mixed together with a kneader or other mixer as mentioned above, relatively gentle agitation is preferred so as not to generate much heat.

For a material set, for an acoustic matching layer, that includes a base resin made of a resin composition including an epoxy resin and surface-treated metal particles and a curing agent for the epoxy resin, the base resin can be prepared by mixing together the epoxy resin and the surface-treated metal particles. When an acoustic matching layer is formed, the base resin and the curing agent are mixed together to obtain the composition according to the present invention. The composition can be formed and cured to form an acoustic matching layer or a precursor sheet thereof.

Acoustic Matching Sheet (Acoustic Matching Layer)

The composition according to the present invention can be formed into a sheet shape and optionally subjected to processing such as cutting or dicing to the desired thickness or shape to obtain an acoustic matching sheet. This acoustic matching sheet is used as an acoustic matching layer for an acoustic wave probe. The structure of an acoustic wave probe including the acoustic matching layer will be described later.

If the composition according to the present invention has a thermoplastic resin as the binder, the composition is heated to thermally melt the resin, is formed into the desired sheet shape, and is solidified by cooling. Thus, an acoustic matching sheet or a precursor sheet thereof can be formed.

Alternatively, if the composition according to the present invention has a thermosetting resin as the binder and optionally a curing agent, the composition is formed into the desired sheet shape in a low-temperature range where no curing reaction occurs or in a low-temperature range where the curing rate is sufficiently low. The sheet is then optionally heated or otherwise treated to form a crosslinked structure, thereby curing the sheet. Thus, an acoustic matching sheet or a precursor sheet thereof is obtained. That is, if a composition having a thermosetting resin as the binder is used, the resulting acoustic matching sheet is a cured product formed from the composition according to the present invention so as to have a three-dimensional network structure.

Acoustic Wave Probe

An acoustic wave probe according to the present invention has the acoustic matching sheet formed from the composition according to the present invention as an acoustic matching layer.

An example configuration of the acoustic wave probe according to the present invention is illustrated in FIG. 1. The acoustic wave probe illustrated in FIG. 1 is an ultrasound probe for an ultrasound diagnostic apparatus. An ultrasound probe is an acoustic wave probe that uses, particularly, ultrasound as an acoustic wave. Thus, the basic structure of an ultrasound probe can be directly applied to an acoustic wave probe.

Ultrasound Probe

An ultrasound probe 10 is one of the main components of an ultrasound diagnostic apparatus and has the functions of generating ultrasound and transmitting and receiving an ultrasound beam. As shown in FIG. 1, the configuration of the ultrasound probe 10 includes, in order from the distal side (the side to be brought into contact with a living body serving as a subject), an acoustic lens 1, an acoustic matching layer 2, a piezoelectric element layer 3, and a backing material 4. Recently, there has also been proposed an ultrasound probe including a stack of an ultrasonic vibrator (piezoelectric element) for transmission and an ultrasonic vibrator (piezoelectric element) for reception that are formed of different materials in order to receive higher-order harmonics.

Piezoelectric Element Layer

The piezoelectric element layer 3 is a portion that generates ultrasound and has electrodes attached to both sides of the piezoelectric element. As a voltage is applied to the piezoelectric element, it vibrates by repeatedly shrinking and expanding, thus generating ultrasound.

As materials for piezoelectric elements, so-called ceramic inorganic piezoelectric materials have been widely used, including single crystals such as those of quartz, $LiNbO_3$, $LiTaO_3$, and $KNbO_3$, thin films such as those of ZnO and AlN, and sintered bodies such as those of $Pb(Zr,Ti)O_3$-based materials that have been subjected to polarization treatment. In general, piezoelectric ceramics such as lead zirconate titanate (PZT) have been used because of their high conversion efficiency.

Piezoelectric elements for detecting received waves on the high-frequency side also need to be sensitive over a wider bandwidth. Accordingly, organic piezoelectric materials using organic polymer materials such as polyvinylidene fluoride (PVDF) have been used for piezoelectric elements suitable for high-frequency, wide-bandwidth applications.

Furthermore, for example, JP2011-071842A discloses a capacitive micromachined ultrasonic transducer (cMUT) based on micro-electro-mechanical system (MEMS) technology, which provides an array structure with good short-pulse characteristics and wide-bandwidth characteristics, high suitability for mass production, and little characteristic variation.

Any of these piezoelectric element materials is preferred for use in the present invention.

Backing Material

The backing material 4 is disposed on the backside of the piezoelectric element layer 3 and suppresses undesirable vibrations to shorten the width of ultrasound pulses, thereby contributing to an improvement in the distance resolution of an ultrasound diagnostic image.

Acoustic Matching Layer

The acoustic matching layer 2 is provided to reduce the difference in acoustic impedance between the piezoelectric element layer 3 and the subject for efficient transmission and reception of ultrasound.

Acoustic Lens

The acoustic lens 1 is provided to focus ultrasound in the slice direction by refraction to improve the resolution. It is desirable that the acoustic lens 1 come into close contact with a living body serving as a subject and allow ultrasound to match the acoustic impedance of the living body (for the human body, 1.4 to 1.7 $\times$ 10$^6$ kg/m$^2$/sec). It is also desirable that the acoustic lens 1 itself exhibit little ultrasound attenuation.

That is, the use of a material in which the sound velocity is sufficiently lower than in the human body, which exhibits little ultrasound attenuation, and whose acoustic impedance is close to that of the human skin as the material for the acoustic lens 1 increases the transmission and reception sensitivity for ultrasound.

The operation of the thus-configured ultrasound probe 10 will be described. As a voltage is applied between the electrodes disposed on both sides of the piezoelectric element, the piezoelectric element layer 3 resonates to transmit an ultrasound signal from the acoustic lens 1 to the subject. Upon reception, the piezoelectric element layer 3 vibrates in response to the signal reflected from the subject (echo signal). This vibration is converted into an electrical signal to obtain an image.

Manufacture of Acoustic Wave Probe

The acoustic wave probe according to the present invention can be produced in a usual manner except that the resin composition for an acoustic matching layer according to the present invention is used. Specifically, a method for manufacturing the acoustic wave probe according to the present invention includes forming an acoustic matching layer on a piezoelectric element using the resin composition for an acoustic matching layer according to the present invention. The piezoelectric element can be provided on a backing material in a usual manner.

An acoustic lens is further formed on the acoustic matching layer in a usual manner using a material for forming the acoustic lens.

Acoustic Wave Measuring Apparatus

An acoustic wave measuring apparatus according to the present invention has the acoustic wave probe according to the present invention. The acoustic wave measuring apparatus has the functions of indicating the signal intensity of a signal received by the acoustic wave probe and converting the signal into an image.

It is also preferred that the acoustic wave measuring apparatus according to the present invention be an ultrasound measuring apparatus including an ultrasound probe.

**Examples**

The present invention will hereinafter be described in more detail with reference to examples in which ultrasound was used as an acoustic wave. The present invention, however, is not limited to ultrasound, but may also be applied to acoustic waves at audible frequencies as long as an appropriate frequency is selected depending on, for example, the subject and the measurement conditions.

Preparation Example 1: Preparation of Resin Compositions for Acoustic Matching Layers

(Binder: Thermoplastic Resin)

Metal particles and thermoplastic resins were mixed together in the amounts shown in the following tables, and the mixtures were kneaded with a twin-screw mixer (the trade name Labo Plastomill Model C available from Toyo Seiki Seisaku-sho, Ltd.). The kneading conditions were as follows: the temperature was 20°C above the melting point of the thermoplastic resin, the screw rotational speed was 15 rpm, and the kneading time was 20 minutes. Thus, resin compositions, for acoustic matching layers, that had a thermoplastic resin as the binder were prepared.

Test Example 1: Viscosity Evaluation

Each of the compositions obtained in Preparation Example 1 was heated to a temperature of 20°C above the melting point of the thermoplastic resin used as the binder for the composition, and the viscosity (Pa·s) at that temperature was measured. The viscosity measurement conditions were as follows. The measured viscosity was rated on the following rating scale to evaluate the handleability of the composition. A lower viscosity indicates a better handleability.

Viscosity Measurement Conditions

Measuring apparatus: cone-type rotational rheometer ("RheoStress RS6000" available from Haake Inc.)
Rotational speed: 1 rpm
Measurement time: 60 seconds

Rating Scale of Viscosity

A: less than 500 Pa·s
B: from 500 Pa s to less than 1,000 Pa·s
C: from 1,000 Pa·s to less than 5,000 Pa·s
D: 5,000 Pa·s or more

The results are shown in the following tables.

Test Example 2: Density

Each of the compositions obtained in Preparation Example 1 was compressionmolded at a temperature of 10°C above the melting point of the thermoplastic resin used as the binder and was then trimmed to form a sheet having a length of 60 mm, a width of 60 mm, and a thickness of 2 mm.
The density of the resulting sheet at 25°C was measured in accordance with the method of density measurement specified as Method A (immersion method) in JIS K 7112 (1999) with an electronic densimeter (the trade name "SD-200L" available from Alfa Mirage Co., Ltd.).
The results are shown in the following tables.

Test Example 3: Mechanical Strength

Each of the compositions obtained in Preparation Example 1 was compressionmolded at a temperature of 10°C above the melting point of the thermoplastic resin used as the binder and was then trimmed to form a sheet having a length of 50 mm, a width of 50 mm, and a thickness of 0.4 mm. This sheet was punched to obtain a strip-shaped test specimen with a length of 40 mm, a width of 5 mm, and a thickness of 0.4 mm. This test specimen was subjected to a tensile test with a tensile tester (the trade name Autograph AGS-X/20N available from Shimadzu Corporation) at a tensile speed of 30 mm/min in the longitudinal direction of the test specimen. The energy required for the test specimen to fracture (fracture energy) was determined and rated on the following rating scale.

Rating Scale of Fracture Energy

A: 50 J or more
B: from 40 J to less than 50 J
C: from 30 J to less than 40 J
D: less than 30 J

The results are shown in the following tables.

Test Example 4: Variation in Acoustic Impedance (AI)

Each of the compositions obtained in Preparation Example 1 was compressionmolded at a temperature of 10°C above the melting point of the thermoplastic resin used as the binder and was then trimmed to form a sheet having a length of 5 cm, a width of 5 cm, and a thickness of 2 mm. The acoustic impedance of the resulting sheet was calculated as the product of the density (in $g/cm^3$) and the sound velocity (in m/sec) (i.e., density $\times$ sound velocity) at a total of five positions, i.e., near the four corners and in the center of the sheet. The standard deviation of the acoustic impedances at the five positions was determined and rated on the following rating scale to evaluate the variation in acoustic characteristics.

Sound Velocity

The ultrasound velocity (in m/sec) was measured in accordance with JIS Z 2353 (2003) at 25°C with a sing-around sound velocity measuring apparatus (the trade name "Type UVM-2" available from Ultrasonic Engineering Co., Ltd.). The ultrasound velocity was measured over the entire area inside a circle with a diameter of 1.5 cm (i.e., the size of a single-channel small probe) at each of the five measurement positions.

Density

The density at 25°C was measured at the five measurement positions in accordance with the method of density measurement specified as Method A (immersion method) in JIS K 7112 (1999) with an electronic densimeter (the trade name "SD-200L" available from Alfa Mirage Co., Ltd.). Here, the density at each measurement position was determined by cutting a 10 mm $\times$ 10 mm square piece of sheet from the sound velocity measurement area (a circle with a diameter of 1.5 cm) and measuring the density (in $g/cm^3$) of the cut piece of sheet (10 mm $\times$ 10 mm square).

Rating Scale of Variation in Acoustic Characteristics

A: standard deviation of less than 0.5
B: standard deviation of from 0.5 to less than 0.7
C: standard deviation of from 0.7 to less than 0.9
D: standard deviation of 0.9 or more

The results are shown in the following tables.

Test Example 5: Corrosion Resistance

Each of the as-prepared compositions obtained in Preparation Example 1 was formed into a sheet shape with a length of 5 cm, a width of 5 cm, and a thickness of 2 mm as in Test Example 4 above. After each sheet was allowed to stand at 85°C and 90% RH for 500 hours in an environmental test chamber, the test specimen was removed therefrom and examined for its surface condition at ten randomly selected positions at 50x magnification under a BS-D8000II microscope (available from Sonic-Group). The examination results were rated on the following scale to evaluate the degree of corrosion.

Rating Scale of Corrosion Resistance

A: No corroded areas were found.
B: No corroded spots were found, but discoloration was observed.
C: One to five corroded spots were observed.
D: Six or more corroded spots were observed, or rust was observed over the entire surface.

The results are shown in the following tables.

Table 1-1

| | | | Example 1 | Example 2 | Example 3 | Example 4 | Example 5 | Example 6 | Example 7 | Example 8 | Example 9 | Example 10 | Example 11* |
|---|---|---|---|---|---|---|---|---|---|---|---|---|---|
| Composition | Metal particles | Type | (A-1) Fe | (A-2) Zn | (A-3) Ti | (A-4) Cu | (A-5) Ni | (A-6) Zr | (A-7) Mo | (A-8) Ag | (A-9) Pt | (A-10) Au | (A-11) Fe |
| | | Surface treatment | Iron phosphate | Iron phosphate | Iron phosphate | Iron phosphate | Iron phosphate | Iron phosphate | Iron phosphate | Iron phosphate | Iron phosphate | Iron phosphate | Phosphoric acid |
| | | Amount (parts by mass) | 100 | 100 | 100 | 100 | 100 | 100 | 100 | 100 | 100 | 100 | 100 |
| | Thermoplastic resin | Type | (B-1) PA6 | (B-1) PA6 | (B-1) PA6 | (B-1) PA6 | (B-1) PA6 | (B-1) PA6 | (B-1) PA6 | (B-1) PA6 | (B-1) PA6 | (B-1) PA6 | (B-1) PA6 |
| | | Amount (parts by mass) | 25 | 25 | 25 | 25 | 25 | 25 | 25 | 25 | 25 | 25 | 25 |
| Evaluation | Viscosity | | A | A | A | A | A | A | A | A | B | B | A |
| | Density (g/cm$^3$) | | 3.7 | 3.5 | 2.9 | 3.8 | 3.8 | 3.4 | 4.0 | 4.0 | 4.8 | 4.7 | 3.7 |
| | Fracture energy | | A | A | A | A | A | B | B | B | C | B | A |
| | Variation in Al | | A | A | A | A | A | A | A | A | A | A | A |
| | Corrosion resistance | | A | B | C | A | A | A | A | A | A | A | B |

* Reference Example

Table 1-2

| Composition | | | Example 12* | Example 13* | Example 14 | Example 15 | Example 16 | Example 17 | Example 18 | Example 19 | Example 20 | Example 21 | Example 22 |
|---|---|---|---|---|---|---|---|---|---|---|---|---|---|
| Composition | Metal particles | Type | (A-12) Fe | (A-13) Fe | (A-14) Fe | (A-1) Fe | (A-1) Fe | (A-1) Fe | (A-1) Fe | (A-1) Fe | (A-1) Fe | (A-1) Fe | (A-1) Fe |
| | | Surface treatment | Zinc phosphate | Manganese phosphate | Silane coupling | Iron phosphate | Iron phosphate | Iron phosphate | Iron phosphate | Iron phosphate | Iron phosphate | Iron phosphate | Iron phosphate |
| | | Amount (parts by mass) | 100 | 100 | 100 | 100 | 100 | 100 | 100 | 100 | 100 | 100 | 100 |
| | Thermoplastic resin | Type | (B-1) PA6 | (B-1) PA6 | (B-1) PA6 | (B-2) HDPE | (B-3) PP | (B-4) GPPS | (B-5) ABS | (B-6) PMMA | (B-7) POM | (B-8) PC | (B-9) PPE |
| | | Amount (parts by mass) | 25 | 25 | 25 | 25 | 25 | 25 | 25 | 25 | 25 | 25 | 25 |
| Evaluation | Viscosity | | A | A | B | A | A | A | A | A | A | A | A |
| | Density (g/cm$^3$) | | 3.7 | 3.7 | 3.7 | 3.2 | 3.0 | 3.3 | 3.3 | 3.6 | 4.0 | 3.6 | 3.6 |
| | Fracture energy | | A | B | B | B | B | B | A | A | A | A | B |
| | Variation in Al | | A | B | C | B | A | A | A | A | A | A | A |
| | Corrosion resistance | | B | A | C | A | A | A | A | A | A | A | A |
| * Reference Examples | | | | | | | | | | | | | |

Table 1-3

| | | | Example 23 | Example 24 | Example 25 | Example 26 | Example 27 | Example 28 | Example 29 | Example 30 | Example 31 | Example 32 | Example 33 | Comparative Example 1 |
|---|---|---|---|---|---|---|---|---|---|---|---|---|---|---|
| Composition | Metal particles | Type | (A-1) Fe | (A-1) Fe | (A-1) Fe | (A-1) Fe | (A-1) Fe | (A-1) Fe | (A-1) Fe | (A-1) Fe | (A-1) Fe | (A-1) Fe | (A-1) Fe | (X-1) Fe |
| | | Surface treatment | Iron phosphate | Iron phosphate | Iron phosphate | Iron phosphate | Iron phosphate | Iron phosphate | Iron phosphate | Iron phosphate | Iron phosphate | Iron phosphate | Iron phosphate | - |
| | | Amount (parts by mass) | 100 | 100 | 100 | 100 | 100 | 100 | 100 | 100 | 100 | 100 | 100 | 100 |
| | Thermoplastic resin | Type | (B-10) PBT | (B-11) PES | (B-12) PEI | (B-13) PPS | (B-14) TPU | (B-15) PVC | (B-16) PVDF | (B-1) PA6 | (B-1) PA6 | (B-1) PA6 | (B-1) PA6 | (B-1) PA6 |
| | | Amount (parts by mass) | 25 | 25 | 25 | 25 | 25 | 25 | 25 | 12 | 18 | 35 | 50 | 25 |
| Evaluation | Viscosity | | A | A | A | A | A | A | A | C | B | A | A | D |
| | Density (g/cm$^3$) | | 4.5 | 3.7 | 3.8 | 3.9 | 3.6 | 3.6 | 4.5 | 4.9 | 4.2 | 3.1 | 2.7 | 3.7 |
| | Fracture energy | | B | A | A | B | A | B | C | A | A | A | A | D |
| | Variation in Al | | B | A | A | B | A | A | C | A | A | A | A | D |
| | Corrosion resistance | | A | A | A | A | A | B | A | A | A | A | A | D |

The details on the metal particles and the thermoplastic resins listed in the tables are set forth below:

Metal Particles

(A-1) Fe (average particle size: 4.1 μm), iron phosphate treatment
(A-2) Zn (average particle size: 6.3 μm), iron phosphate treatment
(A-3) Ti (average particle size: 5.1 μm), iron phosphate treatment
(A-4) Cu (average particle size: 3.6 μm), iron phosphate treatment
(A-5) Ni (average particle size: 2.6 μm), iron phosphate treatment
(A-6) Zr (average particle size: 6.1 μm), iron phosphate treatment
(A-7) Mo (average particle size: 4.4 μm), iron phosphate treatment
(A-8) Ag (average particle size: 5.6 μm), iron phosphate treatment
(A-9) Pt (average particle size: 6.8 μm), iron phosphate treatment
(A-10) Au (average particle size: 3.0 μm), iron phosphate treatment
(A-11) Fe (average particle size: 4.1 μm), phosphoric acid treatment
(A-12) Fe (average particle size: 4.1 μm), zinc phosphate treatment
(A-13) Fe (average particle size: 4.1 μm), manganese phosphate treatment
(A-14) Fe (average particle size: 4.1 μm), 3-methacryloxypropyltrimethoxysilane treatment
(A-15) Fe (average particle size: 4.1 μm), 3-glycidoxypropyltrimethoxysilane treatment
(A-16) Fe (average particle size: 4.1 μm), 2-(3,4-epoxycyclohexyl) ethyltrimethoxysilane treatment
(X-1) Fe (average particle size: 4.1 μm), no surface treatment

Thermoplastic Resins

(B-1) Polyamide 6 (PA6)
(B-2) High-density polyethylene (HDPE)
(B-3) Polypropylene (PP)
(B-4) Polystyrene (GPPS)
(B-5) Acrylonitrile-butadiene-styrene copolymer (ABS)
(B-6) Polymethyl methacrylate (PMMA)
(B-7) Polyacetal (POM)
(B-8) Polycarbonate (PC)
(B-9) Polyphenylene ether (PPE)
(B-10) Polybutylene terephthalate (PBT)
(B-11) Polyethersulfone (PES)
(B-12) Polyetherimide (PEI)
(B-13) Polyphenylene sulfide (PPS)
(B-14) Thermoplastic polyurethane (TPU)
(B-15) Polyvinyl chloride (PVC)
(B-16) Fluorocarbon resin (PVDF)

As shown in Tables 1-1 to 1-3 above, the composition prepared from a combination of metal particles that were not subjected to surface treatment and a thermoplastic resin exhibited high viscosity upon melting of the resin (melt viscosity) and thus had poor handleability. In addition, the sheet formed from this composition had poor mechanical strength and large variation in acoustic characteristics in the sheet and was also susceptible to corrosion (Comparative Example 1).

In contrast, the compositions prepared from combinations of surface-treated metal particles and thermoplastic resins exhibited low viscosity upon melting of the resin (melt viscosity). In addition, the sheets formed from these compositions had good mechanical strength and little variation in acoustic characteristics in the sheets and were also resistant to corrosion (Examples 1 to 33).

Preparation Example 2: Preparation of Resin Compositions for Acoustic Matching Layers

(Binder: Thermosetting Resin in Combination with Curing Agent)

Metal particles, epoxy resins, and curing agents were mixed together in the amounts shown in the following tables. Specifically, the metal particles and the epoxy resins were degassed at room temperature under a reduced pressure of 1.0 Pa with agitation at 1,800 rpm in an agitator (the trade name Awatori Rentaro ARV-310 available from Thinky Corporation) for 4 minutes. To the mixtures were added curing agents, followed by further degassing with agitation under the same conditions.

Thus, resin compositions, for acoustic matching layers, that included a thermosetting resin as the binder were prepared.

Test Example 6: Viscosity

The viscosity (Pa s) of each of the as-prepared compositions obtained in Preparation Example 2 was measured. The viscosity measurement conditions were as follows. The measured viscosity was rated on the following rating scale to evaluate the handleability of the composition.

Viscosity Measurement Conditions

Measuring apparatus: cone-type rotational rheometer ("RheoStress RS6000" available from Haake Inc.)
Measurement temperature: 25°C
Rotational speed: 1 rpm
Measurement time: 60 seconds

Rating Scale of Viscosity

A: less than 500 Pa·s
B: from 500 Pa s to less than 1,000 Pa·s
C: from 1,000 Pa·s to less than 5,000 Pa·s
D: 5,000 Pa·s or more

The results are shown in the following tables.

Test Example 7: Density

Each of the as-prepared compositions obtained in Preparation Example 2 was cast into a sheet shape and was allowed to stand at a temperature of 80°C for 18 hours and then at a temperature of 150°C for 1 hour to form a cured sheet with a length of 60 mm, a width of 60 mm, and a thickness of 2 mm.

The density of the resulting sheet was measured as in Test Example 2.

The results are shown in the following tables.

Test Example 8: Mechanical Strength

Each of the as-prepared compositions obtained in Preparation Example 2 was formed into a sheet shape and was allowed to stand at a temperature of 80°C for 18 hours and then at a temperature of 150°C for 1 hour to form a cured sheet with a length of 50 mm, a width of 50 mm, and a thickness of 0.4 mm. This cured sheet was punched to obtain a strip-shaped test specimen with a length of 40 mm, a width of 5 mm, and a thickness of 0.4 mm. This test specimen was used to evaluate the fracture energy as in Test Example 3.

The results are shown in the following tables.

Test Example 9: Variation in Acoustic Impedance (AI)

Each of the as-prepared compositions obtained in Preparation Example 2 was formed into a sheet shape and was allowed to stand at a temperature of 80°C for 18 hours and then at a temperature of 150°C for 1 hour to form a cured sheet with a length of 5 cm, a width of 5 cm, and a thickness of 2 mm. This cured sheet was used to evaluate the variation in acoustic impedance (AI) as in Test Example 4.

The results are shown in the following tables.

Test Example 10: Corrosion Resistance

Each of the as-prepared compositions obtained in Preparation Example 2 was formed into a sheet shape and was allowed to stand at a temperature of 80°C for 18 hours and then at a temperature of 150°C for 1 hour to form a cured sheet with a length of 5 cm, a width of 5 cm, and a thickness of 2 mm. This cured sheet was used to evaluate the corrosion resistance as in Test Example 5.

The results are shown in the following tables.

Table 2-1

| | | | Example 34 | Example 35 | Example 36 | Example 37 | Example 38 | Example 39 | Example 40 | Example 41 | Example 42 | Example 43 |
|---|---|---|---|---|---|---|---|---|---|---|---|---|
| Composition | Metal particles | Type | (A-1) Fe | (A-2) Zn | (A-3) Ti | (A-4) Cu | (A-5) Ni | (A-6) Zr | (A-7) Mo | (A-8) Ag | (A-9) Pt | (A-10) |
| | | Surface treatment | Iron phosphate | Iron phosphate | Iron phosphate | Iron phosphate | Iron phosphate | Iron phosphate | Iron phosphate | Iron phosphate | Iron phosphate | Iron phosphate |
| | | Amount (parts by mass) | 100 | 100 | 100 | 100 | 100 | 100 | 100 | 100 | 100 | 100 |
| | Epoxy resin | Type | (C-1) | (C-1) | (C-1) | (C-1) | (C-1) | (C-1) | (C-1) | (C-1) | (C-1) | (C-1) |
| | | Amount (parts by mass) | 20 | 20 | 20 | 20 | 20 | 20 | 20 | 20 | 20 | 20 |
| | Curing agent | Type | (D-1) | (D-1) | (D-1) | (D-1) | (D-1) | (D-1) | (D-1) | (D-1) | (D-1) | (D-1) |
| | | Amount (parts by mass) | 8 | 8 | 8 | 8 | 8 | 8 | 8 | 8 | 8 | 8 |
| Evaluation | Viscosity | | A | A | A | A | A | A | A | A | A | A |
| | Density (g/cm$^3$) | | 3.5 | 3.4 | 2.8 | 3.6 | 3.6 | 3.2 | 3.8 | 3.8 | 4.5 | 4.4 |
| | Fracture energy | | A | A | A | A | A | B | B | B | C | B |
| | Variation in Al | | A | A | A | A | A | A | A | A | A | A |
| | Corrosion resistance | | A | B | C | A | A | A | A | A | A | A |

Table 2-2

| | | | Example 44* | Example 45* | Example 46* | Example 47 | Example 48 | Example 49 | Example 50 | Example 51 | Example 52 | Example 53 |
|---|---|---|---|---|---|---|---|---|---|---|---|---|
| Composition | Metal particles | Type | (A-11) Fe | (A-12) Fe | (A-13) Fe | (A-14) Fe | (A-1) Fe | (A-1) Fe | (A-1) Fe | (A-1) Fe | (A-1) Fe | (A-1) Fe |
| | | Surface treatment | Phosphoric acid | Zinc phosphate | Manganese phosphate | Silane coupling | Iron phosphate | Iron phosphate | Iron phosphate | Iron phosphate | Iron phosphate | Iron phosphate |
| | | Amount (parts by mass) | 100 | 100 | 100 | 100 | 100 | 100 | 100 | 100 | 100 | 100 |
| | Epoxy resin | Type | (C-1) | (C-1) | (C-1) | (C-1) | (C-2) | (C-3) | (C-4) | (C-5) | (C-6) | (C-7) |
| | | Amount (parts by mass) | 20 | 20 | 20 | 20 | 20 | 20 | 20 | 20 | 20 | 20 |
| | Curing agent | Type | (D-1) | (D-1) | (D-1) | (D-1) | (D-1) | (D-1) | (D-1) | (D-1) | (D-1) | (D-1) |
| | | Amount (parts by mass) | 8 | 8 | 8 | 8 | 8 | 8 | 8 | 8 | 8 | 8 |
| Evaluation | Viscosity | | A | A | A | B | A | A | A | A | A | A |
| | Density (g/cm$^3$) | | 3.5 | 3.5 | 3.5 | 3.5 | 3.5 | 3.4 | 3.4 | 3.4 | 3.5 | 3.4 |
| | Fracture energy | | A | A | B | B | A | A | A | A | B | B |
| | Variation in Al | | A | A | B | C | A | A | A | A | A | A |
| | Corrosion resistance | | B | B | A | C | A | A | A | A | A | A |
| * Reference Example | | | | | | | | | | | | |

Table 2-3

| | | | Example 54 | Example 55 | Example 56 | Example 57 | Example 58 | Example 59 | Example 60 | Example 61 | Comparative Example 2 |
|---|---|---|---|---|---|---|---|---|---|---|---|
| Composition | Metal particles | Type | (A-1) Fe | (A-1) Fe | (A-1) Fe | (A-1) Fe | (A-1) Fe | (A-1) Fe | (A-1) Fe | (A-1) Fe | (Fe) |
| | | Surface treatment | Iron phosphate | Iron phosphate | Iron phosphate | Iron phosphate | Iron phosphate | Iron phosphate | Iron phosphate | Iron phosphate | - |
| | | Amount (parts by mass) | 100 | 100 | 100 | 100 | 100 | 100 | 100 | 100 | 100 |
| | Epoxy resin | Type | (C-1) | (C-1) | (C-1) | (C-1) | (C-1) | (C-1) | (C-1) | (C-1) | (C-1) |
| | | Amount (parts by mass) | 20 | 20 | 20 | 20 | 10 | 15 | 30 | 50 | 20 |
| | Curing agent | Type | (D-2) | (D-3) | (D-4) | (D-5) | (D-1) | (D-1) | (D-1) | (D-1) | (D-1) |
| | | Amount (parts by mass) | 8 | 8 | 8 | 8 | 4 | 6 | 12 | 20 | 8 |
| Evaluation | Viscosity | | A | A | A | A | C | B | A | A | D |
| | Density (g/cm$^3$) | | 3.5 | 3.5 | 3.4 | 3.4 | 4.6 | 3.9 | 2.9 | 2.3 | 3.3 |
| | Fracture energy | | A | C | B | B | C | B | A | A | D |
| | Variation in Al | | A | B | B | A | A | A | A | A | D |
| | Corrosion resistance | | A | A | A | B | A | A | A | A | D |

The details of the epoxy resins and the curing agents listed in the tables are set forth below:

Epoxy Resins

(C-1) Bisphenol A diglycidyl ether ("jER825" (trade name) available from Mitsubishi Chemical Corporation, epoxy equivalent weight: 170)
(C-2) Bisphenol A diglycidyl ether ("jER828" (trade name) available from Mitsubishi Chemical Corporation, epoxy equivalent weight: 190)
(C-3) Bisphenol A diglycidyl ether ("jER834" (trade name) available from Mitsubishi Chemical Corporation, epoxy equivalent weight: 230)
(C-4) Bisphenol F diglycidyl ether ("EPICLON 830" (trade name) available from DIC Corporation, epoxy equivalent weight: 170)
(C-5) Epoxy novolac resin (Product No. 406775 available from Sigma-Aldrich Company, epoxy equivalent weight: 170)
(C-6) Bisphenol A propoxylate diglycidyl ether (available from Sigma-Aldrich Company, epoxy equivalent weight: 228)
(C-7) 4,4'-Methylenebis(N,N-diglycidylaniline) (available from Tokyo Chemical Industry Co., Ltd., epoxy equivalent weight: 106)

Curing Agents

(D-1) Polyamidoamine ("LUCKAMIDE EA-330" available from DIC Corporation)
(D-2) Triethylenetetramine (reagent available from Tokyo Chemical Industry Co., Ltd.)
(D-3) 2,4,6-Tris(dimethylaminomethyl)phenol (the trade name "LUVEAK DMP-30" available from Nacalai Tesque, Inc.)
(D-4) 2-Ethyl-4-methylimidazole (reagent available from Tokyo Chemical Industry Co., Ltd.)
(D-5) Hexahydrophthalic anhydride ("RIKACID HH" available from New Japan Chemical Co., Ltd.)

As shown in Tables 2-1 to 2-3 above, the composition prepared from a combination of metal particles that were not subjected to surface treatment and a thermosetting resin exhibited high viscosity and thus had poor handleability. In addition, the sheet formed from this composition had poor mechanical strength and large variation in acoustic characteristics in the sheet and was also susceptible to corrosion (Comparative Example 2).

In contrast, the compositions prepared from combinations of surface-treated metal particles and thermosetting resins exhibited low viscosity. In addition, the sheets formed from these compositions had good mechanical strength and little variation in acoustic characteristics in the sheets and were also resistant to corrosion (Examples 34 to 61).

Preparation Example 3: Preparation of Resin Compositions for Acoustic Matching Layers

Resin compositions for acoustic matching layers were prepared from the ingredients shown in the following tables as in the above preparation examples.

Test Example 11: Ultrasound Sensitivity

Each of the as-prepared compositions obtained in Preparation Example 3 was formed into a sheet with a length of 100 mm, a width of 100 mm, and a thickness of 2 mm as in the above test examples.

A sinusoidal signal (one wave) with a frequency of 5 MHz output from an ultrasonic oscillator (e.g., the trade name "FG-350" function generator available from Iwatsu Electric Co., Ltd.) was input to an ultrasound probe (available from Japan Probe Co., Ltd.) so that the ultrasound probe generated an ultrasound pulse wave with a center frequency of 5 MHz in water. The amplitudes of the resulting ultrasound before and after propagation through the sheet were measured with an ultrasonic receiver (e.g., the trade name "VP-5204A" oscilloscope available from Panasonic Corporation) at a water temperature of 25°C. The acoustic wave (ultrasound) sensitivity was compared to compare the acoustic wave (ultrasound) attenuation of each sheet.

The ultrasound sensitivity was given by the following equation:

$$(\text{ultrasound sensitivity}) = 20 \times \log(\text{Vs/Vin})$$

where Vin is the peak voltage of an input wave with a half-width of 50 nsec or less from the ultrasonic oscillator, and Vs is the voltage obtained when the acoustic wave (ultrasound) generated in response to the voltage Vin was received by the ultrasonic oscillator after propagation through the sheet and reflection by the opposing surface of the sheet.

The resulting ultrasound sensitivity was rated on the following scale to evaluate the sensitivity.

Rating Scale of Sensitivity

A: -66 dB or more
B: from -70 dB to less than -66 dB
C: less than -70 dB

The results are shown in the following table. The following table also shows the results of evaluation for the density, fracture energy, standard deviation of AI (variation in AI), and corrosion resistance measured as in the above test examples.

Table 3

| | | | Example 1 | Example 14 | Example 62 | Example 63 | Example 34 | Example 47 | Example 64 | Example 65 |
|---|---|---|---|---|---|---|---|---|---|---|
| Composition | Metal particles | Type | (A-1) Fe | (A-14) Fe | (A-15) Fe | (A-16) Fe | (A-1) Fe | (A-14) Fe | (A-15) Fe | (A-16) Fe |
| | | Surface treatment | Iron phosphate | Silane coupling | Silane coupling | Silane coupling | Iron phosphate | Silane coupling | Silane coupling | Silane coupling |
| | | Amount (parts by mass) | 100 | 100 | 100 | 100 | 100 | 100 | 100 | 100 |
| | Thermoplastic resin | Type | (B-1) PA6 | (B-1) PA6 | (B-1) PA6 | (B-1) PA6 | - | - | - | - |
| | | Amount (parts by mass) | 25 | 25 | 25 | 25 | - | - | - | - |
| | Epoxy resin | Type | - | - | - | - | (C-1) | (C-1) | (C-1) | (C-1) |
| | | Amount (parts by mass) | - | - | - | - | 20 | 20 | 20 | 20 |
| | Curing agent | Type | - | - | - | - | (D-1) | (D-1) | (D-1) | (D-1) |
| | | Amount (parts by mass) | - | - | - | - | 8 | 8 | 8 | 8 |
| Evaluation | Viscosity | | A | B | B | B | A | B | B | B |
| | Density (g/cm$^3$) | | 3.7 | 3.7 | 3.7 | 3.7 | 3.5 | 3.5 | 3.5 | 3.5 |
| | Fracture energy | | A | B | B | B | A | B | B | B |
| | Variation in Al | | A | C | B | C | A | C | B | C |
| | Corrosion resistance | | A | C | B | C | A | C | B | C |
| | Ultrasound sensitivity | | B | A | A | A | B | A | A | A |

EP 3 706 436 B1

As shown in Table 3, the use of metal particles subjected to silane coupling agent treatment was found to be effective in increasing the sensitivity for acoustic waves.

This application claims priority to JP2017-212208, filed in Japan on November 1, 2017.

**Reference Signs List**

1    acoustic lens
2    acoustic matching layer (acoustic matching sheet)
3    piezoelectric element layer
4    backing material
7    housing
9    cord
10   ultrasound probe

**Claims**

1.  A resin composition for an acoustic matching layer, comprising

    a binder, and
    surface-treated metal particles,
    **characterized in that**
    the surface-treated metal particles are metal particles having been subjected to surface treatment with a silane coupling agent or a phosphoric acid compound that includes iron phosphate,
    wherein the surface-treated metal particles have a particle size of 1 to 10 $\mu$m, and
    wherein the binder includes a thermoplastic resin or a thermosetting resin, and the thermosetting resin includes an epoxy resin, a phenolic resin, a melamine resin, a urea resin, an unsaturated polyester resin, an alkyd resin, a thermosetting polyurethane resin, or a thermosetting polyimide resin.

2.  The resin composition for an acoustic matching layer according to claim 1, wherein the surface-treated metal particles include at least one metal in Groups 4 to 12 of the periodic table,

    wherein the metal in Groups 4 to 12 of the periodic table is preferably a metal in Period 4 of the periodic table, and
    wherein the surface-treated metal particles more preferably include at least one of iron, copper, or nickel.

3.  The resin composition for an acoustic matching layer according to any one of claims 1 to 2,
    wherein the thermoplastic resin includes at least one of a polyamide resin, an acrylonitrile-butadiene-styrene copolymer, a meth(acrylic) resin, a polyacetal resin, a polycarbonate resin, a polyethersulfone resin, a polyetherimide resin, or a thermoplastic polyurethane resin.

4.  The resin composition for an acoustic matching layer according to any one of claims 1 to 2,
    wherein the binder includes an epoxy resin and a curing agent for the epoxy resin, wherein the epoxy resin preferably includes at least one of a bisphenol A epoxy resin, a bisphenol F epoxy resin, or a phenol novolac epoxy resin, and wherein the curing agent more preferably includes at least one of a primary amine or a secondary amine.

5.  A cured product formed from the resin composition for an acoustic matching layer according to claim 4.

6.  An acoustic matching sheet comprising the cured product according to claim 5.

7.  An acoustic matching sheet formed from the resin composition for an acoustic matching layer according to any one of claims 1 to 4.

8.  An acoustic wave probe comprising the acoustic matching sheet according to claim 6 or 7 as an acoustic matching layer.

9.  An acoustic wave measuring apparatus comprising the acoustic wave probe according to claim 8.

10. The acoustic wave measuring apparatus according to claim 9, wherein the acoustic wave measuring apparatus is an ultrasound diagnostic apparatus.

11. A method for manufacturing an acoustic wave probe, comprising forming an acoustic matching layer on a piezoelectric element using the resin composition for an acoustic matching layer according to any one of claims 1 to 4.

12. Use of a resin composition according to any of claims 1 to 4 for forming an acoustic matching layer.

**Patentansprüche**

1. Harzzusammensetzung für eine akustische Anpassungsschicht, umfassend

   ein Bindemittel und
   oberflächenbehandelte Metallpartikel,
   **dadurch gekennzeichnet, dass**
   die oberflächenbehandelten Metallpartikel Metallpartikel sind, die einer Oberflächenbehandlung mit einem Silan-Kupplungsmittel oder einer Phosphorsäureverbindung, die Eisenphosphat umfasst, unterzogen worden sind, worin die oberflächenbehandelten Metallpartikel eine Partikelgröße von 1 bis 10 $\mu$m aufweisen und
   worin das Bindemittel ein thermoplastisches Harz oder ein warmhärtendes Harz umfasst und das warmhärtende Harz ein Epoxyharz, ein Phenolharz, ein Melaminharz, ein Harnstoffharz, ein ungesättigtes Polyesterharz, ein Alkydharz, ein warmhärtendes Polyurethanharz oder ein warmhärtendes Polyimidharz umfasst.

2. Harzzusammensetzung für eine akustische Anpassungsschicht gemäß Anspruch 1, worin die oberflächenbehandelten Metallpartikel mindestens ein Metall der Gruppen 4 bis 12 des Periodensystems umfassen,

   worin das Metall der Gruppen 4 bis 12 des Periodensystems bevorzugt ein Metall in Periode 4 des Periodensystems ist und
   worin die oberflächenbehandelten Metallpartikel stärker bevorzugt zumindest eines von Eisen, Kupfer oder Nickel umfassen.

3. Harzzusammensetzung für eine akustische Anpassungsschicht gemäß irgendeinem der Ansprüche 1 bis 2, worin das thermoplastische Harz mindestens eines von einem Polyamidharz, einem Acrylnitril-Butadien-StyrolCopolymer, einem Meth(acryl)Harz, einem Polyacetalharz, einem Polycarbonatharz, einem Polyethersulfonharz, einem Polyetherimidharz oder einem thermoplastischen Polyurethanharz umfasst.

4. Harzzusammensetzung für eine akustische Anpassungsschicht gemäß irgendeinem der Ansprüche 1 bis 2, worin das Bindemittel ein Epoxyharz und ein Härtungsmittel für das Epoxyharz umfasst, worin das Epoxyharz bevorzugt zumindest eines von einem Bisphenol A-Epoxyharz, einem Bisphenol F-Epoxyharz oder einem Phenol-Novolak-Epoxyharz umfasst, und worin das Härtungsmittel stärker bevorzugt zumindest eines von einem primären Amin oder einem sekundären Amin umfasst.

5. Gehärtetes Produkt, gebildet aus der Harzzusammensetzung für eine akustische Anpassungsschicht gemäß Anspruch 4.

6. Akustisches Anpassungsblatt, umfassend das gehärte Produkt gemäß Anspruch 5.

7. Akustisches Anpassungsblatt, gebildet aus der Harzzusammensetzung für eine akustische Anpassungsschicht gemäß irgendeinem der Ansprüche 1 bis 4.

8. Schallwellensonde, umfassend das akustische Anpassungsblatt gemäß Anspruch 6 oder 7 als eine akustische Anpassungsschicht.

9. Schallwellen-Messvorrichtung, umfassend die Schallwellensonde gemäß Anspruch 8.

10. Schallwellen-Messvorrichtung gemäß Anspruch 9, worin die Schallwellen-Messvorrichtung eine Ultraschall-Diagnosevorrichtung ist.

**11.** Verfahren zur Herstellung einer Schwallwellensonde, umfassend das Bilden einer akustischen Anpassungsschicht auf einem piezoelektrischen Element unter Verwendung der Harzzusammensetzung für eine akustische Anpassungsschicht gemäß irgendeinem der Ansprüche 1 bis 4.

**12.** Verwendung einer Harzzusammensetzung gemäß irgendeinem der Ansprüche 1 bis 4 zum Bilden einer akustischen Anpassungsschicht.

**Revendications**

1. Composition de résine pour une couche d'adaptation acoustique, comprenant

    un liant, et
    des particules métalliques traitées en surface,
    **caractérisée en ce que**
    les particules métalliques traitées en surface sont des particules métalliques ayant été soumises à un traitement de surface avec un agent de couplage de type silane ou un composé d'acide phosphorique qui inclut le phosphate de fer,
    dans laquelle les particules métalliques traitées en surface présentent une taille de particule de 1 à 10 $\mu$m, et
    dans laquelle le liant comprend une résine thermoplastique ou une résine thermodurcissable, et la résine thermodurcissable comprend une résine époxy, une résine phénolique, une résine de mélamine, une résine d'urée, une résine de polyester insaturé, une résine alkyde, une résine de polyuréthane thermodurcissable, ou une résine de polyimide thermodurcissable.

2. Composition de résine pour une couche d'adaptation acoustique selon la revendication 1, dans laquelle

    les particules métalliques traitées en surface comprennent au moins un métal des groupes 4 à 12 du tableau périodique,
    dans laquelle le métal des groupes 4 à 12 du tableau périodique est de préférence un métal de la période 4 du tableau périodique, et
    dans laquelle les particules métalliques traitées en surface comprennent de manière davantage préférée au moins l'un parmi le fer, le cuivre ou le nickel.

3. Composition de résine pour une couche d'adaptation acoustique selon l'une quelconque des revendications 1 à 2, dans laquelle la résine thermoplastique comprend au moins l'un parmi une résine de polyamide, un copolymère d'acrylonitrile-butadiène-styrène, une résine méth(acrylique), une résine de polyacétal, une résine de polycarbonate, une résine de polyéthersulfone, une résine de polyétherimide, ou une résine thermoplastique de polyuréthane.

4. Composition de résine pour une couche d'adaptation acoustique selon l'une quelconque des revendications 1 à 2, dans laquelle le liant comprend une résine époxy et un agent de durcissement pour la résine époxy, dans laquelle la résine époxy comprend de préférence au moins l'une parmi une résine époxy de bisphénol A, une résine époxy de bisphénol F, ou une résine époxy de phénol novolaque, et dans laquelle l'agent de durcissement comprend de manière davantage préférée au moins l'une parmi une amine primaire ou une amine secondaire.

5. Produit durci formé à partir de la composition de résine pour une couche d'adaptation acoustique selon la revendication 4.

6. Feuille d'adaptation acoustique comprenant le produit durci selon la revendication 5.

7. Feuille d'adaptation acoustique formée à partir de la composition de résine pour une couche d'adaptation acoustique selon l'une quelconque des revendications 1 à 4.

8. Sonde d'onde acoustique comprenant la feuille d'adaptation acoustique selon la revendication 6 ou la revendication 7 en tant que couche d'adaptation acoustique.

9. Appareil de mesure d'onde acoustique comprenant la sonde d'onde acoustique selon la revendication 8.

10. Appareil de mesure d'onde acoustique selon la revendication 9, dans lequel l'appareil de mesure d'onde acoustique est un appareil de diagnostic à ultrasons.

11. Procédé de fabrication d'une sonde d'onde acoustique, comprenant la formation d'une couche d'adaptation acoustique sur un élément piézoélectrique en utilisant la composition de résine pour une couche d'adaptation acoustique selon l'une quelconque des revendications 1 à 4.

12. Utilisation d'une composition de résine selon l'une quelconque des revendications 1 à 4 pour former une couche d'adaptation acoustique.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017012436 A
- JP 2013192113 A
- US 2017252465 A1
- JP 2009296055 A
- US 2016338666 A1
- JP H0462007 A
- JP 2001200169 A
- EP 2004064 A2
- JP 2011071842 A
- JP 2017212208 A

**Non-patent literature cited in the description**

- *Journal of the Surface Finishing Society of Japan,* 2010, vol. 61 (3), 216
- *Journal of the Surface Finishing Society of Japan,* 2013, vol. 64 (12), 640